# FASCICULE DE BREVET EUROPEEN

(11) **EP 3 515 544 B1**
(45) Date de publication et mention de la délivrance du brevet: **15.12.2021**
(21) Numéro de dépôt: 17781160.1
(22) Date de dépôt: 19.09.2017
(51) Int. Cl.: A61M 39/22, A61M 39/10, A61M 5/14, A61M 39/24

(54) **DISPOSITIF DE PURGE POUR SYSTÈME D'ADMINISTRATION DE FLUIDES DE TRAITEMENT MÉDICAL**
SPÜLVORRICHTUNG FÜR EIN SYSTEM ZUR VERABREICHUNG MEDIZINISCHER BEHANDLUNGSFLÜSSIGKEITEN
PURGE DEVICE FOR A SYSTEM FOR ADMINISTERING MEDICAL TREATMENT FLUIDS

(30) Priorité: 26.09.2016 FR 1659014
(43) Date de publication de la demande: 31.07.2019
(73) Titulaire: Doran International, 69780 Toussieu (FR)
(72) Inventeur: BUISSON, Philippe, 69780 Toussieu (FR); FIORE, Brice, 67800 Bischheim (FR)
(74) Mandataire: Germain Maureau
(86) Numéro de dépôt international: PCT/FR2017/052498
(87) Numéro de publication internationale: WO 2018/055274

(56) Documents cités:
- WO-A1-2007/033319
- WO-A1-2016/037648
- FR-A1- 2 581 315
- US-A1- 2003 181 850
- US-A1- 2006 047 249

## Description

La présente invention concerne un dispositif de purge pour système d'administration de fluides de traitement médical.

Le document EP2217305 divulgue un système d'administration de fluides de traitement médical, comprenant notamment :
- un boîtier pourvu de plusieurs éléments de connexion sur chacun desquels un récipient peut être connecté, et comprenant en outre plusieurs conduits de passage de liquide, chaque conduit de passage de liquide comportant une portion d'extrémité proximale reliée fluidiquement à un élément de connexion respectif et portion d'extrémité distale opposée à la portion d'extrémité proximale respective,
- un tube d'écoulement contenant plusieurs conduits d'écoulement de fluide sensiblement parallèles et s'étendant sur toute la longueur du tube d'écoulement, le tube d'écoulement comportant une portion d'extrémité distale destinée à être connectée à un cathéter et une portion d'extrémité proximale raccordée au boîtier de telle sorte que chaque conduit d'écoulement de fluide du tube d'écoulement est relié fluidiquement à un conduit de passage de fluide.

Une telle configuration du système d'administration décrit dans le document EP2217305 permet d'administrer à un patient simultanément divers liquides de traitement médical sans mélange préalable de ces liquides au sein du boîtier et du tube d'écoulement de fluide, et donc de limiter les réactions médicamenteuses et la formation de précipités au sein du système d'administration donc de contrôler avec précision l'administration de chaque liquide. Ces dispositions permettent plus particulièrement de contrôler avec précision la quantité administrée et le débit d'administration de chaque liquide.

L'inconvénient d'un tel système d'administration réside toutefois dans le fait que pour purger une voie d'administration formée par un conduit de passage de fluide et le conduit d'écoulement de fluide respectif (par exemple lorsque l'on souhaite remplacer une solution médicamenteuse par une autre), il est nécessaire de prévoir un robinet entre le tube d'écoulement et le cathéter. Or, l'utilisation d'un tel robinet pour purger une voie d'administration du système d'administration précité implique soit de stopper les autres voies d'administration du système d'administration, soit de purger également les autres voies d'administration et donc de perdre les solutions médicamenteuses qui se trouvent dans ces autres voies d'administration. En outre, la présence d'un tel robinet est susceptible d'induire un risque de mélange des différentes solutions médicamenteuses lors de leur passage à travers le robinet.

Le document FR2581315 divulgue un dispositif de purge selon le préambule de la revendication 1.

La présente invention vise à remédier à ces inconvénients.

Le problème technique à la base de l'invention consiste donc à fournir un dispositif de purge qui soit de structure simple et économique, tout en permettant la purge d'un conduit d'écoulement de fluide du tube d'écoulement sans stopper le traitement du patient et sans risque de perte des solutions médicamenteuses contenues dans les autres conduits d'écoulement de fluide du tube d'écoulement.

A cet effet, la présente invention concerne un dispositif de purge selon la revendication 1.

Une telle configuration de l'élément de raccordement et de l'élément d'obturation permet de pouvoir aisément purger un conduit d'écoulement de fluide d'un tube d'écoulement, et ce tout simplement en déplaçant l'élément d'obturation dans une position d'obturation dans laquelle la partie d'obturation obture l'extrémité distale du canal d'écoulement de fluide relié au conduit d'écoulement de fluide à purger. Dans une telle position d'obturation, le fluide à purger s'écoule à travers l'ouverture d'écoulement de fluide associée au canal d'écoulement de fluide obturée par la partie d'obturation et l'ouverture d'évacuation de fluide.

En outre, étant donné que, dans chaque position d'obturation, la partie d'obturation est configurée pour obturer l'extrémité distale que d'un seul canal d'écoulement de fluide, la purge d'un conduit d'écoulement de fluide à l'aide du dispositif de purge selon la présente invention n'a aucun impact sur les autres conduits d'écoulement de fluide.

Par conséquent, le dispositif de purge selon la présente invention permet la purge d'un conduit d'écoulement de fluide d'un tube d'écoulement sans stopper le traitement du patient, et sans risque de perte des solutions médicamenteuses contenues dans les autres conduits d'écoulement de fluide du tube d'écoulement.

Le dispositif de purge peut en outre présenter une ou plusieurs des caractéristiques suivantes, prises seules ou en combinaison.

Selon un mode de réalisation de l'invention, dans chaque position d'obturation occupée par l'élément d'obturation, l'ouverture d'évacuation de fluide est disposée en regard d'une ouverture d'écoulement de fluide respective.

Selon un mode de réalisation de l'invention, l'orifice de sortie de l'embout de connexion est relié fluidiquement aux extrémités distale de l'ensemble des canaux d'écoulement de fluide lorsque l'élément d'obturation est dans l'au moins une position de libération, et est relié fluidiquement aux extrémités distale des canaux d'écoulement de fluide qui ne sont pas obturées par la partie d'obturation lorsque l'élément d'obturation est dans l'une quelconque des positions d'obturation.

Selon un mode de réalisation de l'invention, l'élément d'obturation est monté mobile en rotation par rapport à l'élément de raccordement.

Selon un mode de réalisation de l'invention, l'élément d'obturation est monté mobile en rotation autour d'un axe de rotation qui est sensiblement coïncident avec l'axe longitudinal de l'élément d'obturation. En d'autres termes, l'élément d'obturation est monté mobile en rotation autour de son axe longitudinal.

Selon un mode de réalisation de l'invention, le corps creux a une forme globalement tubulaire.

Selon un mode de réalisation de l'invention, le passage traversant axial est globalement cylindrique.

Selon un mode de réalisation de l'invention, l'ouverture d'évacuation de fluide débouche radialement dans le passage traversant axial.

Selon un mode de réalisation de l'invention, l'élément de raccordement comprend une partie de montage s'étendant au moins en partie dans le passage traversant axial du corps creux, la partie de montage de l'élément de raccordement étant pourvue des ouvertures d'écoulement de fluide.

Selon un mode de réalisation de l'invention, la partie d'obturation est un doigt d'obturation.

Selon un mode de réalisation de l'invention, la partie d'obturation s'étend à partir d'une extrémité distale du corps creux.

Selon un mode de réalisation de l'invention, la partie d'obturation s'étend radialement vers l'intérieur du corps creux.

Selon un mode de réalisation de l'invention, chaque ouverture d'écoulement de fluide débouche dans une surface périphérique externe de l'élément de raccordement.

Selon un mode de réalisation de l'invention, la surface périphérique externe de l'élément de raccordement est globalement cylindrique.

Selon un mode de réalisation de l'invention, la partie de montage de l'élément de raccordement est délimitée extérieurement par la surface périphérique externe dans laquelle débouche chaque ouverture d'écoulement de fluide.

Selon un mode de réalisation de l'invention, chaque ouverture d'écoulement de fluide s'étend radialement.

Selon un mode de réalisation de l'invention, les ouvertures d'écoulement de fluide sont décalées angulairement les unes des autre par rapport à un axe longitudinal de l'élément de raccordement.

Selon un mode de réalisation de l'invention, les ouvertures d'écoulement de fluide s'étendent dans un même plan d'extension.

Selon un mode de réalisation de l'invention, les ouvertures d'écoulement de fluide sont régulièrement décalées angulairement les unes des autre par rapport à l'axe longitudinal de l'élément de raccordement

Selon un mode de réalisation de l'invention, les canaux d'écoulement de fluide s'étendent sensiblement parallèlement.

Selon un mode de réalisation de l'invention, l'embout de connexion comprend un conduit de refoulement destiné à être relié à un récipient de récupération, le conduit de refoulement étant relié fluidiquement à l'ouverture d'évacuation de fluide prévue sur l'élément d'obturation. Il convient d'être noté que le récipient de récupération peut être tout type de récipient, et par exemple une poche souple.

Selon un mode de réalisation de l'invention, le conduit de refoulement est équipé d'un clapet anti-retour configuré pour autoriser un écoulement de fluide uniquement de l'ouverture d'évacuation de fluide vers le récipient de récupération.

Selon un mode de réalisation de l'invention, l'embout de connexion comprend une partie de montage dans laquelle est monté au moins en partie l'élément d'obturation.

Selon un mode de réalisation de l'invention, le corps creux est au moins en partie monté dans la partie de montage de l'embout de connexion.

Selon un mode de réalisation de l'invention, la partie de montage de l'embout de connexion est globalement cylindrique.

Selon un mode de réalisation de l'invention, le conduit de refoulement s'étend à partir d'une surface périphérique extérieure de la partie de montage, et par exemple radialement vers l'extérieur à partir de la surface périphérique extérieure de la partie de montage.

Selon un mode de réalisation de l'invention, la partie de montage de l'embout de connexion comporte une surface interne pourvue d'une gorge annulaire, le conduit de refoulement et l'ouverture d'évacuation de fluide débouchant dans la gorge annulaire.

Selon un mode de réalisation de l'invention, l'embout de connexion délimite au moins en partie une chambre interne dans laquelle débouchent les extrémités distales des canaux d'écoulement de fluide, l'orifice de sortie de l'embout de connexion débouchant dans la chambre interne. La chambre interne peut par exemple être délimitée au moins en partie par l'élément de raccordement et l'embout de connexion.

Selon un mode de réalisation de l'invention, l'embout de connexion comprend une partie de connexion tronconique destinée à être connectée au cathéter, la partie de connexion tronconique étant reliée fluidiquement à l'orifice de sortie.

Selon un mode de réalisation de l'invention, l'embout de connexion est du type Luer ou Luer Lock.

Selon un mode de réalisation de l'invention, l'embout de connexion comprend une partie de connexion filetée intérieurement destinée à être connectée au cathéter, la partie de connexion filetée intérieurement entourant la partie de connexion tronconique.

Selon un mode de réalisation de l'invention, l'élément de raccordement comprend une surface d'extrémité distale, par exemple sensiblement plane, dans laquelle débouchent les extrémités distales des canaux d'écoulement de fluide. Avantageusement, la surface d'extrémité distale délimite au moins en partie la chambre interne.

Selon un mode de réalisation de l'invention, l'élément de raccordement comprend une partie de raccordement configurée pour recevoir, par exemple par emboîtement à force, la portion d'extrémité distale du tube d'écoulement.

Selon un mode de réalisation de l'invention, l'élément d'obturation comporte une partie d'appui configurée pour coopérer avec la partie de montage de l'embout de connexion de manière à limiter la profondeur d'introduction de l'élément d'obturation dans la partie de montage. La partie d'appui peut comprendre une surface d'appui, par exemple annulaire, configurée pour coopérer avec une extrémité proximale de la partie de montage de l'embout de connexion.

Selon un mode de réalisation de l'invention, l'élément d'obturation comporte une partie de butée configurée pour coopérer avec l'élément de raccordement de manière à limiter la profondeur d'introduction de l'élément de raccordement dans l'élément d'obturation, et plus particulièrement à limiter la profondeur d'introduction de l'élément de raccordement dans le passage traversant axial du corps creux. La partie de butée peut comprendre une surface de butée, par exemple annulaire, configurée pour coopérer avec l'élément de raccordement, et par exemple avec la partie de raccordement de l'élément de raccordement.

Selon un mode de réalisation de l'invention, l'élément de raccordement comporte une rainure annulaire dans laquelle est reçue une portion d'extrémité proximale du corps creux. La rainure annulaire peut par exemple est prévue sur la partie de raccordement de l'élément de raccordement, et débouchée axialement en direction de l'élément d'obturation.

Selon un mode de réalisation de l'invention, l'élément de raccordement comprend une pluralité de broches de raccordement, chaque broche de raccordement étant creuse et étant destinée à être engagée dans une portion d'extrémité distale d'un conduit d'écoulement de fluide respectif du tube d'écoulement. Avantageusement, la partie de raccordement comprend les broches de raccordement.

Selon un mode de réalisation de l'invention, l'extrémité proximale de chaque canal d'écoulement de fluide débouche dans l'extrémité libre d'une broche de raccordement respective.

Selon un mode de réalisation de l'invention, l'élément de raccordement comprend un conduit d'écoulement de fluide central, et les canaux d'écoulement de fluide sont répartis, par exemple régulièrement répartis, autour du conduit d'écoulement de fluide central.

Selon un mode de réalisation de l'invention, l'élément de raccordement et l'embout de connexion sont montés fixes l'un par rapport à l'autre.

Selon un mode de réalisation de l'invention, au moins l'un de l'élément de raccordement, l'élément d'obturation et l'embout de connexion est en matière plastique, et par exemple en matière plastique transparente.

Selon un mode de réalisation de l'invention, l'élément d'obturation est configuré pour occuper une pluralité de positions de libération dans chacune desquelles la partie d'obturation libère les extrémités distales des canaux d'écoulement de fluide et l'ouverture d'évacuation de fluide est isolée fluidiquement des ouvertures d'écoulement de fluide.

Selon un mode de réalisation de l'invention, le dispositif de purge pourrait comprendre des moyens de détection configurés pour détecter le passage de l'organe d'obturation dans une position d'obturation, et par exemple d'une position de libération à une position d'obturation, et des moyens d'avertissement configurés pour émettre un signal d'avertissement, par exemple sonore ou visuel, lorsque l'organe d'obturation est maintenu dans une position d'obturation pendant une durée de temps supérieure à une valeur de seuil prédéterminée. Le signal d'avertissement peut également être transmis à un opérateur, par exemple par Wifi ou Bluetooth. Ces dispositions permettent d'éviter de maintenir par erreur ou par oubli l'élément d'obturation dans une position d'obturation, et donc d'assurer une administration sécurisée des différents fluides.

Selon un mode de réalisation de l'invention, le dispositif de purge pourrait comprendre une pluralité d'éléments de raccordement et une pluralité d'éléments d'obturation, chaque élément d'obturation étant associé à un élément de raccordement respectif, et chaque élément de raccordement étant destiné à être raccordé à une portion d'extrémité distale d'un tube d'écoulement respectif.

De toute façon l'invention sera bien comprise à l'aide de la description qui suit en référence aux dessins schématiques annexés représentant, à titre d'exemple non limitatif, une forme d'exécution de ce dispositif de purge.
Figure 1 est une vue en perspective d'un dispositif de purge selon l'invention.
Figure 2 est une vue éclatée en perspective du dispositif de purge de la figure 1.
Figure 3 est une vue en couple longitudinale du dispositif de purge de la figure 1.
Figure 4 est une vue en perspective d'un élément de raccordement du dispositif de purge de la figure 1.
Figure 5 est une vue en perspective de l'élément de raccordement de la figure 4 inséré dans un élément d'obturation du dispositif de purge de la figure 1.
Figure 6 est une vue en perspective d'un embout de connexion du dispositif de purge de la figure 1.

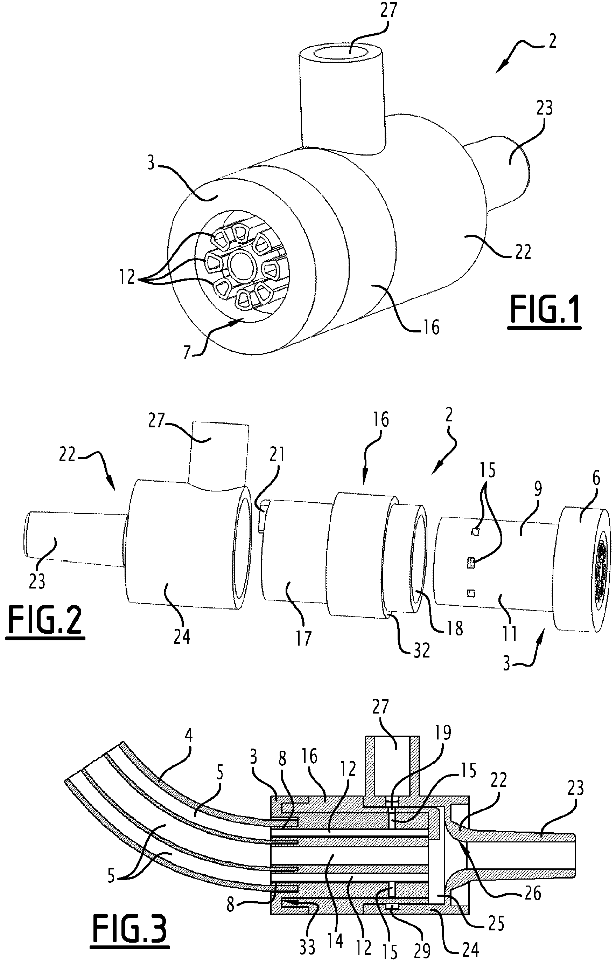

Les figures 1 à 6 représentent un dispositif de purge 2 pour un système d'administration de fluides de traitement médical, et par exemple pour un système d'administration tel que décrit dans le document EP2217305.

Comme montré sur les figures 2 à 5, le dispositif de purge 2 comprend un élément de raccordement 3 destiné à être raccordé à une portion d'extrémité distale d'un tube d'écoulement 4 appartenant à un système d'administration de fluides de traitement médical et comportant une pluralité de conduits d'écoulement de fluide 5. L'élément de raccordement 3 peut par exemple être réalisé en matière plastique.

L'élément de raccordement 3 comprend plus particulièrement une partie de raccordement 6 comportant un logement de réception 7 configuré pour recevoir, par emboîtement à force, la portion d'extrémité distale du tube d'écoulement 4. Selon le mode de réalisation représenté sur les figures, le logement de réception 7 est globalement cylindrique et débouche dans une face d'extrémité proximale 3.1 de l'élément de raccordement 3. La partie de raccordement 6 comprend en outre une pluralité de broches de raccordement 8 s'étendant dans le logement de réception 7 et sensiblement parallèlement les unes par rapport aux autres. Chaque broche de raccordement 8 est creuse et est destinée à être engagée dans une portion d'extrémité distale d'un conduit d'écoulement de fluide 5 respectif du tube d'écoulement 4.

L'élément de raccordement 3 comprend en outre une partie de montage 9 délimitée extérieurement par une surface périphérique externe 11 qui est globalement cylindrique et qui s'étend sensiblement coaxialement avec le logement de réception 7.

L'élément de raccordement 3 comprend de plus une pluralité de canaux d'écoulement de fluide 12 s'étendant sur sensiblement toute la longueur de l'élément de raccordement 3. Chaque canal d'écoulement de fluide 12 comprend une extrémité proximale 12.1 débouchant dans une extrémité libre d'une broche de raccordement 8 respective et donc destinée à être reliée fluidiquement à un conduit d'écoulement de fluide 5 respectif du tube d'écoulement 4, et une extrémité distale 12.2 opposée à l'extrémité proximale 12.1 respective et débouchant dans une surface d'extrémité distale 13, par exemple sensiblement plane, de la partie de montage 9. Avantageusement, les canaux d'écoulement de fluide 12 s'étendent sensiblement parallèlement.

Selon le mode de réalisation représenté sur les figures, l'élément de raccordement 3 comprend également un conduit d'écoulement de fluide central 14 autour duquel sont régulièrement répartis les canaux d'écoulement de fluide 12. Avantageusement, le conduit d'écoulement de fluide central 14 comprend une extrémité proximale 14.1 débouchant dans l'extrémité libre d'une broche de raccordement 8 respective et donc destinée à être reliée fluidiquement à un conduit d'écoulement de fluide 5 respectif du tube d'écoulement 4, et une extrémité distale 14.2 opposée à l'extrémité proximale 14.1 respective et débouchant dans la surface d'extrémité distale 13 de la partie de montage 8.

Comme montré notamment sur la figure 2, l'élément de raccordement 3 comprend une pluralité d'ouvertures d'écoulement de fluide 15 ménagées sur la partie de montage 9. Chaque ouverture d'écoulement de fluide 15 est reliée fluidiquement à un canal d'écoulement de fluide 12 respectif, et débouche dans la surface périphérique externe 11 de la partie de montage 8. Selon le mode de réalisation représenté sur les figures, les ouvertures d'écoulement de fluide 15 s'étendent radialement dans un même plan d'extension, et sont régulièrement décalées angulairement les unes des autre par rapport à un axe longitudinal de l'élément de raccordement 3.

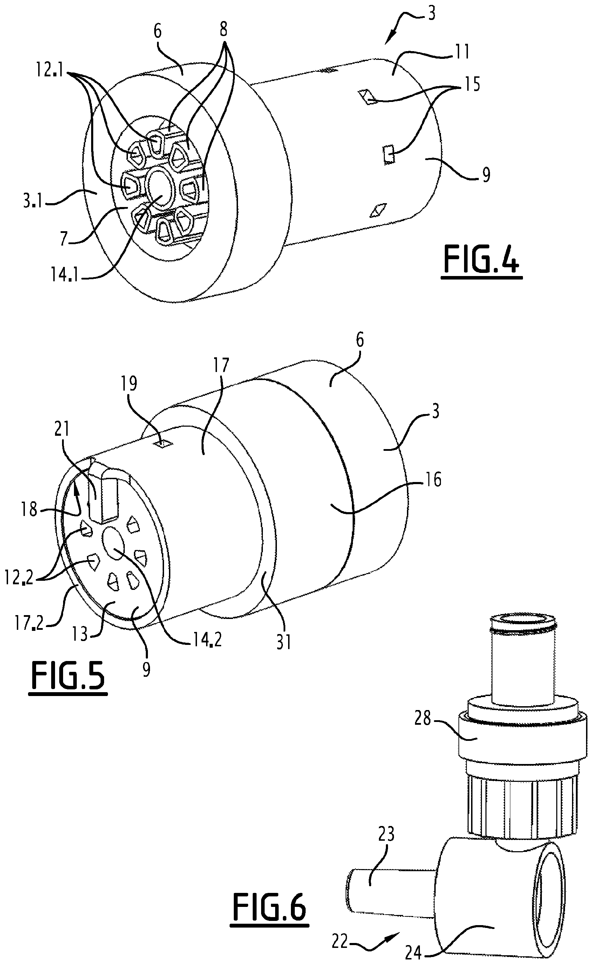

Comme montré plus particulièrement sur les figures 2 et 5, le dispositif de purge 2 comprend en outre un élément d'obturation 16 qui peut par exemple être réalisé en matière plastique. L'élément d'obturation 16 comporte un corps creux 17 ayant une forme globalement tubulaire et s'étendant selon un axe longitudinal. Le corps creux 17 est pourvu d'un passage traversant axial 18 globalement cylindrique et s'étendant sur toute la longueur du corps creux 17. Le passage traversant axial 18 présente ainsi une section transversale sensiblement constante sur toute sa longueur.

Comme cela ressort plus particulièrement des figures 3 et 5, la partie de montage 9 de l'élément de raccordement 3 s'étend dans le passage traversant axial 18, et la surface périphérique externe 11 de la partie de montage 9 présente un diamètre correspondant sensiblement au diamètre interne du passage traversant axial 18. Il en résulte que l'élément d'obturation 16 est monté mobile en rotation par rapport à l'élément de raccordement 3 autour d'un axe de rotation coïncident avec les axes longitudinaux respectivement de l'élément d'obturation 16 et de l'élément de raccordement 3.

L'élément d'obturation 16 comprend en outre une ouverture d'évacuation de fluide 19 prévue sur le corps creux 17 et débouchant dans le passage traversant axial 18, et une partie d'obturation 21, par exemple ayant la forme d'un doigt d'obturation, s'étendant radialement vers l'intérieur à partir d'une extrémité distale 17.2 du corps creux 17.

L'élément d'obturation 16 est plus particulièrement configuré pour occuper une pluralité de positions d'obturation décalées angulairement les unes des autres et dans chacune desquelles la partie d'obturation 21 obture l'extrémité distale 12.2 d'un canal d'écoulement de fluide 12 respectif et l'ouverture d'évacuation de fluide 19 est située en regard de l'ouverture d'écoulement de fluide 15 qui est reliée fluidiquement au canal d'écoulement de fluide 12 dont l'extrémité distale 12.2 est obturée par la partie d'obturation 21. Ainsi, dans chaque position d'obturation, l'ouverture d'évacuation de fluide 19 est reliée fluidiquement à l'ouverture d'écoulement de fluide 15 associée au canal d'écoulement de fluide 12 dont l'extrémité distale 12.2 est obturée par la partie d'obturation 21.

L'élément d'obturation 16 est en outre configuré pour occuper une pluralité de positions de libération décalées angulairement les unes des autres et dans chacune desquelles la partie d'obturation 21 libère les extrémités distale 12.2 des canaux d'écoulement de fluide 12 et l'ouverture d'évacuation de fluide 19 est isolée fluidiquement des ouvertures d'écoulement de fluide 15.

Comme montré plus particulièrement sur les figures 2 et 6, le dispositif de purge 2 comprend en outre un embout de connexion 22 monté fixe par rapport à l'élément de raccordement 3 et destiné à être connecté à un cathéter, et qui peut par exemple être du type Luer ou Luer Lock. Selon le mode de réalisation représenté sur les figures, l'embout de connexion 22 est du type Luer et comporte une partie de connexion tronconique 23 destinée à être connectée au cathéter. L'embout de connexion 22 pourrait cependant être du type Luer Lock et ainsi comprendre en outre une partie de connexion filetée intérieurement et entourant la partie de connexion tronconique 23.

L'embout de connexion 22 comprend en outre une partie de montage 24 qui est tubulaire et globalement cylindrique, et dans laquelle est monté partiellement le corps creux 17. Avantageusement, le diamètre intérieur de la partie de montage 24 correspond sensiblement au diamètre extérieur du corps creux 17. Selon le mode de réalisation représenté sur les figures, la partie de montage 24 de l'embout de connexion 22 et la partie de montage 9 de l'élément de raccordement 3 délimitent une chambre interne 25 dans laquelle débouchent les extrémités distales 12.2 des canaux d'écoulement de fluide 12 et dans laquelle s'étend la partie d'obturation 21.

L'embout de connexion 22 comprend de plus un orifice de sortie 26 débouchant dans la chambre interne 25 et relié fluidiquement à la partie de connexion tronconique 23.

L'embout de connexion 22 comprend également un conduit de refoulement 27 destiné à être relié à un récipient de récupération (non représenté sur les figures), tel qu'une poche souple ou tout autre type de récipient. Le conduit de refoulement 27 est relié fluidiquement à l'ouverture d'évacuation de fluide 19 prévue sur l'élément d'obturation 16, et est équipé d'un clapet anti-retour 28 configuré pour autoriser un écoulement de fluide uniquement de l'ouverture d'évacuation de fluide 19 vers le récipient de récupération. Selon le mode de réalisation représenté sur les figures, le conduit de refoulement 27 s'étend radialement vers l'extérieur à partir de la surface périphérique extérieure de la partie de montage 24, et la surface intérieure de la partie de montage 24 comporte une pourvue d'une gorge annulaire 29 s'étendant autour du corps creux 17 et dans laquelle débouchent le conduit de refoulement 27 et l'ouverture d'évacuation de fluide 19.

Comme montré plus particulièrement sur les figures 3 et 5, l'élément d'obturation comporte une surface d'appui annulaire 31 configurée pour coopérer avec une extrémité proximale de la partie de montage 24 de l'embout de connexion 22 de manière à limiter la profondeur d'introduction du corps creux 17 dans la partie de montage 24, et une surface de butée annulaire 32 configurée pour coopérer avec la partie de raccordement 6 de l'élément de raccordement 3 de manière à limiter la profondeur d'introduction de la partie de montage 9 de l'élément de raccordement 3 dans le passage traversant axial 18 du corps creux 17.

En outre, selon le mode de réalisation représenté sur les figures, l'élément de raccordement 3 comporte une rainure annulaire 33 dans laquelle est reçue une portion d'extrémité proximale du corps creux 17. La rainure annulaire 33 peut par exemple est prévue sur la partie de raccordement 6 de l'élément de raccordement 3, et débouchée axialement en direction de l'élément d'obturation 16.

Un procédé de purge d'un conduit d'écoulement de fluide 5 d'un tube d'écoulement 4 appartenant à un système d'administration de fluides de traitement médical à l'aide du dispositif de purge 2 selon la présente invention va maintenant être décrit.

Un tel précédé de purge comprend les étapes suivantes :
- raccorder la partie de raccordement 6 de l'élément de raccordement 3 à une portion d'extrémité distale du tube d'écoulement 4,
- connecter la partie de connexion tronconique 23 de l'embout de connexion 22 à un cathéter,
- positionner l'élément d'obturation 16 dans une position d'obturation dans laquelle la partie d'obturation 21 obture l'extrémité distale 12.2 du canal d'écoulement de fluide 12 dont l'extrémité proximale 12.1 est reliée fluidiquement au conduit d'écoulement de fluide 5 à purger,
- purger le conduit d'écoulement de fluide 5 à purger via l'ouverture d'écoulement de fluide 15 qui est reliée fluidiquement au canal d'écoulement de fluide 12 dont l'extrémité distale 12.2 est obturée par la partie d'obturation 21, l'ouverture d'évacuation de fluide 19 et le conduit de refoulement 27,
- récupérer le fluide purgé hors du conduit d'écoulement de fluide 5 à purger dans un récipient de récupération relié au conduit de refoulement 27, un retour de fluide vers l'élément de raccordement 3 étant empêché par le clapet anti-retour 28.

Il convient d'être noté que l'étape de positionnement de l'élément d'obturation 16 peut être réalisée aisément par exemple en prévoyant d'une part un repère de lecture sur l'élément d'obturation 16 et d'autre part une pluralité de repères de positionnement sur l'élément de raccordement 3, chaque repère de positionnement correspondant à une position d'obturation prédéterminée de l'élément d'obturation 16, c'est-à-dire à l'obturation de l'extrémité distale 12.2 d'un canal d'écoulement de fluide 12 prédéterminé.

Selon un mode de réalisation de l'invention, le dispositif de purge pourrait comprendre des moyens de détection configurés pour détecter le passage de l'organe d'obturation d'une position de libération à une position d'obturation, et pour émettre un signal d'avertissement, par exemple sonore ou visuel, lorsque l'organe d'obturation est maintenu dans une position d'obturation pendant une durée de temps supérieure à une valeur de seuil prédéterminée.

Comme il va de soi, l'invention ne se limite pas à la seule forme d'exécution de ce dispositif de purge, décrite ci-dessus à titre d'exemple, elle en embrasse au contraire toutes les variantes de réalisation.

## Revendications

1. Dispositif de purge (2) pour système d'administration de fluides de traitement médical, comprenant :
- un élément de raccordement (3) destiné à être raccordé à une portion d'extrémité distale d'un tube d'écoulement (4) comportant une pluralité de conduits d'écoulement de fluide (5), l'élément de raccordement (3) comprenant :
- une pluralité de canaux d'écoulement de fluide (12), chaque canal d'écoulement de fluide (12) comprenant une extrémité proximale (12.1) destinée à être reliée fluidiquement à un conduit d'écoulement de fluide respectif (5) du tube d'écoulement (4) et une extrémité distale (12.2) opposée à l'extrémité proximale respective (12.1), et
- une pluralité d'ouvertures d'écoulement de fluide (15), chaque ouverture d'écoulement de fluide (15) étant reliée fluidiquement à un canal d'écoulement de fluide (12) respectif,
- un élément d'obturation (16) comprenant une ouverture d'évacuation de fluide (19) et une partie d'obturation (21), l'élément d'obturation (16) étant monté mobile par rapport à l'élément de raccordement (3) et étant configuré pour occuper une pluralité de positions d'obturation dans chacune desquelles la partie d'obturation (21) obture l'extrémité distale (12.2) d'un canal d'écoulement de fluide (12) et l'ouverture d'évacuation de fluide (19) est reliée fluidiquement à l'ouverture d'écoulement de fluide (15) qui est reliée fluidiquement au canal d'écoulement de fluide (12) dont l'extrémité distale est obturée par la partie d'obturation (21)
**caractérisé en ce que** l'élément d'obturation (16) comprend un corps creux (17) s'étendant selon un axe longitudinal et pourvu d'un passage traversant axial (18) s'étendant sur toute la longueur du corps creux (17), l'élément de raccordement (3) s'étendant au moins en partie dans le passage traversant axial (18), **en ce que** l'ouverture d'évacuation de fluide (19) est prévue sur le corps creux (17) et débouche dans le passage traversant axial (18), **en ce que** l'élément d'obturation (16) est configuré pour occuper au moins une position de libération dans laquelle la partie d'obturation (21) libère les extrémités distale (12.2) des canaux d'écoulement de fluide (12) et l'ouverture d'évacuation de fluide (19) est isolée fluidiquement des ouvertures d'écoulement de fluide (15), et **en ce que** le dispositif de purge comprend en outre un embout de connexion (22) destiné à être connecté à un cathéter, l'embout de connexion (22) comprenant un orifice de sortie (26) configuré pour être relié fluidiquement aux extrémités distale (12.2) des canaux d'écoulement de fluide (12) et destiné à être relié fluidiquement au cathéter.

2. Dispositif de purge (2) selon la revendication 1, dans lequel l'élément d'obturation (16) est monté mobile en rotation par rapport à l'élément de raccordement (3).

3. Dispositif de purge (2) selon la revendication 1 ou 2, dans lequel la partie d'obturation (21) s'étend à partir d'une extrémité distale du corps creux (17).

4. Dispositif de purge (2) selon l'une quelconque des revendications 1 à 3, dans lequel la partie d'obturation (21) s'étend radialement vers l'intérieur du corps creux (17).

5. Dispositif de purge (2) selon l'une quelconque des revendications 1 à 4, dans lequel chaque ouverture d'écoulement de fluide (15) débouche dans une surface périphérique externe de l'élément de raccordement (3).

6. Dispositif de purge (2) selon l'une quelconque des revendications 1 à 5, dans lequel les ouvertures d'écoulement de fluide (15) sont décalées angulairement les unes des autre par rapport à un axe longitudinal de l'élément de raccordement (3).

7. Dispositif de purge (2) selon l'une quelconque des revendications 1 à 6, dans lequel l'embout de connexion (22) comprend un conduit de refoulement (27) destiné à être relié à un récipient de récupération, le conduit de refoulement (27) étant relié fluidiquement à l'ouverture d'évacuation de fluide (19) prévue sur l'élément d'obturation (16).

8. Dispositif de purge (2) selon la revendication 7, dans lequel le conduit de refoulement (27) est équipé d'un clapet anti-retour (28) configuré pour autoriser un écoulement de fluide uniquement de l'ouverture d'évacuation de fluide (19) vers le récipient de récupération.

9. Dispositif de purge (2) selon l'une quelconque des revendications 1 à 8, dans lequel l'embout de connexion (22) comprend une partie de montage (24) dans laquelle est monté au moins en partie l'élément d'obturation (16).

10. Dispositif de purge (2) selon l'une quelconque des revendications 1 à 9, dans lequel l'embout de connexion (22) délimite au moins en partie une chambre interne (25) dans laquelle débouchent les extrémités distales (12.2) des canaux d'écoulement de fluide (12), l'orifice de sortie (26) de l'embout de connexion (22) débouchant dans la chambre interne (25).

11. Dispositif de purge (2) selon l'une quelconque des revendications 1 à 10, dans lequel l'embout de connexion (22) comprend une partie de connexion tronconique (23) destinée à être connectée au cathéter, la partie de connexion tronconique (23) étant reliée fluidiquement à l'orifice de sortie (26).

12. Dispositif de purge (2) selon l'une quelconque des revendications 1 à 11, dans lequel l'élément de raccordement (3) comprend une pluralité de broches de raccordement (8), chaque broche de raccordement (8) étant creuse et étant destinée à être engagée dans une portion d'extrémité distale d'un conduit d'écoulement de fluide respectif (5) du tube d'écoulement (4).

13. Dispositif de purge (2) selon l'une quelconque des revendications 1 à 12, lequel comprend des moyens de détection configurés pour détecter le passage de l'organe d'obturation dans une position d'obturation, et des moyens d'avertissement configurés pour émettre un signal d'avertissement lorsque l'organe d'obturation est maintenu dans une position d'obturation pendant une durée de temps supérieure à une valeur de seuil prédéterminée.

## Patentansprüche

1. Spülvorrichtung (2) für ein System zur Verabreichung medizinischer Behandlungsflüssigkeiten, umfassend:
- ein Anschlusselement (3), das zum Anschließen an einen distalen Endabschnitt eines Strömungsrohrs (4) bestimmt ist, das eine Vielzahl von Fluidströmungsleitungen (5) aufweist, wobei das Anschlusselement (3) Folgendes umfasst:
- eine Vielzahl von Fluidströmungskanälen (12), wobei jeder Fluidströmungskanal (12) ein proximales Ende (12.1), das zum fluidischen Verbinden mit einer jeweiligen Fluidströmungsleitung (5) des Strömungsrohrs (4) bestimmt ist, und ein dem jeweiligen proximalen Ende (12.1) gegenüberliegendes distales Ende (12.2) umfasst, und
- eine Vielzahl von Fluidströmungsöffnungen (15), wobei jede Fluidströmungsöffnung (15) mit einem jeweiligen Fluidströmungskanal (12) fluidisch verbunden ist,
- ein Verschlusselement (16), das eine Fluidauslassöffnung (19) und einen Verschlussteil (21) umfasst, wobei das Verschlusselement (16) im Verhältnis zum Anschlusselement (3) beweglich angebracht ist und ausgelegt ist, um eine Vielzahl von Verschlusspositionen einzunehmen, in denen in jeder der Verschlussteil (21) das distale Ende (12.2) eines Fluidströmungskanals (12) verschließt und die Fluidauslassöffnung (19) mit der Fluidströmungsöffnung (15) fluidisch verbunden ist, die mit dem Fluidströmungskanal (12) fluidisch verbunden ist, dessen distales Ende vom Verschlussteil (21) verschlossen ist,
**dadurch gekennzeichnet, dass** das Verschlusselement (16) einen Hohlkörper (17) aufweist, der sich gemäß einer Längsachse erstreckt und mit einem axialen Durchgang (18) versehen ist, der sich über die gesamte Länge des Hohlkörpers (17) erstreckt, wobei sich das Anschlusselement (3) mindestens teilweise im axialen Durchgang (18) erstreckt, und dass die Fluidauslassöffnung (19) auf dem Hohlkörper (17) vorgesehen ist und in den axialen Durchgang (18) ausmündet, dass das Verschlusselement (16) ausgelegt ist, um mindestens eine Freigabeposition einzunehmen, in welcher der Verschlussteil (21) die distalen Enden (12.2) der Fluidströmungskanäle (12) freigibt und die Fluidauslassöffnung (19) von den Fluidströmungsöffnungen (15) fluidisch isoliert ist, und dass die Spülvorrichtung ferner eine Verbindungsspitze (22) umfasst, die zur Verbindung mit einem Katheter bestimmt ist, wobei die Verbindungsspitze (22) eine Ausgangsöffnung (26) umfasst, die ausgelegt ist, um mit den distalen Enden (12.2) der Fluidströmungskanäle (12) fluidisch verbunden zu sein und bestimmt ist, mit dem Katheter fluidisch verbunden zu sein.

2. Spülvorrichtung (2) nach Anspruch 1, wobei das Verschlusselement (16) im Verhältnis zum Anschlusselement (3) rotatorisch beweglich angebracht ist.

3. Spülvorrichtung (2) nach Anspruch 1 oder 2, wobei sich der Verschlussteil (21) ab einem distalen Ende des Hohlkörpers (17) erstreckt.

4. Spülvorrichtung (2) nach einem der Ansprüche 1 bis 3, wobei sich der Verschlussteil (21) radial in das Innere des Hohlkörpers (17) erstreckt.

5. Spülvorrichtung (2) nach einem der Ansprüche 1 bis 4, wobei jede Fluidströmungsöffnung (15) in eine externe periphere Oberfläche des Anschlusselements (3) ausmündet.

6. Spülvorrichtung (2) nach einem der Ansprüche 1 bis 5, wobei die Fluidströmungsöffnungen (15) im Verhältnis zu einer Längsachse des Anschlusselements (3) winklig zueinander versetzt sind.

7. Spülvorrichtung (2) nach einem der Ansprüche 1 bis 6, wobei die Verbindungsspitze (22) eine Auslassleitung (27) umfasst, die bestimmt ist, mit einem Rückgewinnungsbehälter verbunden zu sein, wobei die Auslassleitung (27) mit der auf dem Verschlusselement (16) vorgesehenen Fluidauslassöffnung (19) fluidisch verbunden ist.

8. Spülvorrichtung (2) nach Anspruch 7, wobei die Auslassleitung (27) mit einem Rückschlagventil (28) ausgestattet ist, das ausgelegt ist, um ein Strömen von Fluid nur aus der Fluidauslassöffnung (19) in den Rückgewinnungsbehälter zu gestatten.

9. Spülvorrichtung (2) nach einem der Ansprüche 1 bis 8, wobei die Verbindungsspitze (22) einen Montageteil (24) umfasst, in dem das Verschlusselement (16) mindestens teilweise angebracht ist.

10. Spülvorrichtung (2) nach einem der Ansprüche 1 bis 9, wobei die Verbindungsspitze (22) mindestens teilweise eine innere Kammer (25) begrenzt, in die die distalen Enden (12.2) der Fluidströmungskanäle (12) ausmünden, wobei die Ausgangsöffnung (26) der Verbindungsspitze (22) in die innere Kammer (25) ausmündet.

11. Spülvorrichtung (2) nach einem der Ansprüche 1 bis 10, wobei die Verbindungsspitze (22) einen kegelstumpfförmigen Verbindungsteil (23) umfasst, der bestimmt ist, mit dem Katheter verbunden zu sein, wobei der kegelstumpfförmige Verbindungsteil (23) mit der Ausgangsöffnung (26) fluidisch verbunden ist.

12. Spülvorrichtung (2) nach einem der Ansprüche 1 bis 11, wobei das Anschlusselement (3) eine Vielzahl von Anschlussstiften (8) umfasst, wobei jeder Anschlussstift (8) hohl ist und bestimmt ist, in einen distalen Endabschnitt einer jeweiligen Fluidströmungsleitung (5) des Strömungsrohrs (4) eingesetzt zu sein.

13. Spülvorrichtung (2) nach einem der Ansprüche 1 bis 12, die Detektionsmittel umfasst, die ausgelegt sind, um den Durchgang des Verschlussorgans in einer Verschlussposition zu ermitteln, und Warnmittel, die ausgelegt sind, um ein Warnsignal zu senden, wenn das Verschlussorgan während einer Zeitdauer in einer Verschlussposition gehalten wird, die einen vorher festgelegten Grenzwert überschreitet.

## Claims

1. A purge device (2) for a medical treatment fluids administration system, comprising:
- a coupling element (3) intended to be coupled to a distal end portion of a flow tube (4) including a plurality of fluid flow conduits (5), the coupling element (3) comprising:
- a plurality of fluid flow channels (12), each fluid flow channel (12) comprising a proximal end (12.1) intended to be fluidly connected to a respective fluid flow conduit (5) of the flow tube (4) and a distal end (12.2) opposite to the respective proximal end (12.1), and
- a plurality of fluid flow apertures (15), each fluid flow aperture (15) being fluidly connected to a respective fluid flow channel (12),
- a closure element (16) comprising a fluid evacuation aperture (19) and a closure portion (21), the closure element (16) being movably mounted relative to the coupling element (3) and being configured to occupy a plurality of closed positions in each of which the closure portion (21) closes off the distal end (12.2) of a fluid flow channel (12) and the fluid evacuation aperture (19) is fluidly connected to the fluid flow aperture (15) which is fluidly connected to the fluid flow channel (12) whose distal end is closed off by the closure portion (21),
**characterized in that** the closure element (16) includes a hollow body (17) extending along a longitudinal axis and provided with an axial through passage (18) extending over the entire length of the hollow body (17), the coupling element (3) extending at least partly in the axial through passage (18), **in that** the fluid evacuation aperture (19) is provided on the hollow body (17) and opens into the axial through passage (18), **in that** the closure element (16) is configured to occupy at least one released position in which the closure portion (21) releases the distal ends (12.2) of the fluid flow channels (12) and the fluid evacuation aperture (19) is fluidly isolated from the fluid flow apertures (15), and **in that** the purge device (2) further comprises a connection end fitting (22) intended to be connected to a catheter, the connection end fitting (22) comprising an outlet orifice (26) configured to be fluidly connected to the distal ends (12.2) of the fluid flow channels (12) and intended to be fluidly connected to the catheter.

2. The purge device (2) according to claim 1, wherein the closure element (16) is mounted movable in rotation relative to the coupling element (3).

3. The purge device (2) according to claim 1 or 2, wherein the closure portion (21) extends from a distal end of the hollow body (17).

4. The purge device (2) according to any of claims 1 to 3, wherein the closure portion (21) extends radially towards the inside of the hollow body (17).

5. The purge device (2) according to any one of claims 1 to 4, wherein each fluid flow aperture (15) opens into an outer peripheral surface of the coupling element (3).

6. The purge device (2) according to any one of claims 1 to 5, wherein the fluid flow apertures (15) are angularly offset from each other relative to a longitudinal axis of the coupling element (3).

7. The purge device (2) according to any one of claims 1 to 6, wherein the connection end fitting (22) comprises a discharge conduit (27) intended to be connected to a recovery container, the discharge conduit (27) being fluidly connected to the fluid evacuation aperture (19) provided on the closure element (16).

8. The purge device (2) according to claim 7, wherein the discharge conduit (27) is equipped with a check-valve (28) configured to enable a flowing of a fluid only from the fluid evacuation aperture (19) to the recovery container.

9. The purge device (2) according to any one of claims 1 to 8, wherein the connection end fitting (22) comprises a mounting portion (24) in which the closure element (16) is at least partly mounted.

10. The purge device (2) according to any one of claims 1 to 9, wherein the connection end fitting (22) at least in partly delimits an inner chamber (25) into which the distal ends (12.2) of the fluid flow channels (12) open, the outlet orifice (26) of the connection end fitting (22) opening into the inner chamber (25).

11. The purge device (2) according to any one of claims 1 to 10, wherein the connection end fitting (22) comprises a frustoconical connecting portion (23) intended to be connected to the catheter, the frustoconical connecting portion (23) being fluidly connected to the outlet orifice (26).

12. The purge device (2) according to any one of claims 1 to 11, wherein the coupling element (3) comprises a plurality of coupling pins (8), each coupling pin (8) being hollow and being intended to be engaged in a distal end portion of a respective fluid flow conduit (5) of the flow tube (4).

13. The purge device (2) according to any one of claims 1 to 12, which comprises detection means configured to detect the passage of the closure member into a closed position, and warning means configured to emit a warning signal when the closure member is maintained in a closed position for a period of time greater than a predetermined threshold value.
